(19)

**Europäisches Patentamt**
**European Patent Office**
**Office européen des brevets**

(11) **EP 4 552 628 A1**

(12) **EUROPEAN PATENT APPLICATION**

(43) Date of publication:
**14.05.2025   Bulletin 2025/20**

(21) Application number: **23208205.7**

(22) Date of filing: **07.11.2023**

(51) International Patent Classification (IPC):
**A61H 1/02** *(2006.01)*      **G06F 3/01** *(2006.01)*
**B25J 13/02** *(2006.01)*

(52) Cooperative Patent Classification (CPC):
**A61H 1/0288; A61B 5/1114; A61B 5/1121;**
**A61B 5/1125; A61B 5/225; A61B 5/6806;**
**A61B 5/6824; A61B 5/6825; A61B 5/6826;**
**G06F 3/014; G16H 20/30; G16H 40/63;**
**G16H 40/67; G16H 50/50; G16H 80/00;**      (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicants:
• **University of Ioannina**
  **45110 Ioannina (GR)**
• **Physioloft**
  **14564 Kifisia (GR)**
• **National Technical University of Athens**
  **15773 Zografou (GR)**
• **University of the Peloponnese**
  **22131 Tripoli (GR)**
• **Ostracon Ltd**
  **16777 Ellinikon (GR)**

(72) Inventors:
• **FOTIADIS, Dimitrios**
  **45110 Ioannina (GR)**
• **POTSIKA, Vassiliki**
  **45500 Ioannina (GR)**
• **TACHOS, Nikolaos**
  **45500 Ioannina (GR)**
• **PARDALIS, Athanasios**
  **45332 Ioannina (GR)**
• **PAPAIOANNOU, Christoforos**
  **45332 Ioannina (GR)**
• **MITSIS, Alexandros**
  **45500 Ioannina (GR)**

• **BITHAVAS, Panagiotis**
  **45332 Ioannina (GR)**
• **PAPAGIANNIS, George**
  **15127 Melissia (GR)**
• **TRIANTAFILLOU, Athanasios**
  **14562 Kifisia (GR)**
• **MATSOPOULOS, George**
  **15773 Zografou (GR)**
• **KOURIS, Ioannis**
  **15344 Gerakas (GR)**
• **ECONOMOPOULOS, Theodoros**
  **16233 Vyronas (GR)**
• **SYRINGAS, Pantelis**
  **12134 Peristeri (GR)**
• **TSELIKAS, Nikolaos**
  **22131 Tripoli (GR)**
• **ZESTAS, Orestis**
  **22131 Tripoli (GR)**
• **SOUMIS, Dimitrios**
  **18539 Piraeus (GR)**
• **KYRIAKOU, Kyriakos-Ioannis**
  **15126 Marousi (GR)**
• **SEKLOU, Kyriaki**
  **22131 Tripoli (GR)**
• **PROTOPAPPAS, Vasileios**
  **45500 Ioannina (GR)**
• **MALIZOS, Nikolaos**
  **16777 Ellinikon (GR)**

(74) Representative: **Dodou, Evdokia**
  **Katsimpiri 3**
  **15561 Cholargos, Athens (GR)**

(54) **SYSTEM FOR HAND REHABILITATION COMPRISING HAND EXOSKELETAL DEVICE, SENSOR GLOVE AND GRAPHICAL USER INTERFACE**

(57)   System for the rehabilitation of patients with upper limb spasticity due to acquired brain injury or neurodegenerative diseases, said system comprising a sensor glove designed to capture the range of motion and/or muscle resistance of the affected hand; a hand exoskeletal device comprising a plurality of servomotors, each configured to cause the independent motion of a finger of the affected hand; a user interface that processes sensor data and further comprises an exercise management environment that includes a plurality of predefined exercise routines; and an online dashboard that enables the patient/physician to monitor the results

**(Cont. next page)**

of the exercises performed by the patient. Said system combines sensor technology, real-time monitoring and analysis, rehabilitation games and an online dashboard to provide a comprehensive and engaging rehabilitation experience for patients with upper limb spasticity. Said system has been designed to be able to be used both in clinically controlled environment as well as for self-rehabilitation at patients' home.

**FIG 5**

(52) Cooperative Patent Classification (CPC): (Cont.)
A61H 1/0285; A61H 2201/165; A61H 2201/5007;
A61H 2201/5012; A61H 2201/5043;
A61H 2201/5061; A61H 2201/5064;
A61H 2201/5069; A61H 2201/5084;
A61H 2201/5097; A63B 21/00178; A63B 23/16;
A63B 2024/0096; A63B 2071/065; A63B 2220/10;
A63B 2220/51; A63B 2220/803; A63B 2220/833;
A63B 2225/50

# Description

## TECHNICAL FIELD

The present invention relates to a system for rehabilitation of upper limb spasticity, particularly suitable for patients with spasticity after stroke. It also relates to a method for performing self-rehabilitation of upper limb spasticity using said system.

## BACKGROUND OF THE INVENTION

Spasticity is a symptom highly prevalent in patients with movement disorders due to acquired brain injury such as stroke or neurodegenerative diseases such as multiple sclerosis. It is commonly defined as velocity-dependent hyper-excitability of the muscles to stretch, characterized by exaggerated tendon reflexes, increased resistance to passive movement, and neuronal inhibitory control. Spasticity is experienced as a result of upper motor neuron lesions and includes weakness, loss of dexterity, decreased motor control and reduced endurance. Also, it causes substantially increased dependency in activities of daily living, thereby increasing the need of care, sleep problems and several other difficulties. The financial burden is also significant. Early recognition of spasticity is important to provide adequate treatment and increase the possibility of having better outcomes.

The recovery of hand function is one of the most challenging aspects in stroke rehabilitation. Effective upper limb rehabilitation requires intensive, repetitive, activity-dependent learning. In clinical practice, the main rehabilitation approach is the application of a stretching program. The most common and maximum possible frequency of the program is once or twice daily by a physiotherapist who recommends and trains the patients or their relatives to apply it several times a day. This approach requires the presence of the patients in a specialized physiotherapy center every day, which causes additional difficulty for them and their families, given the reduced motor function of patients with spasticity. However, despite intense therapy, spasticity frequently inhibits active wrist and finger extension, prohibiting any attainment of new functional capacity. Also, traditional methods for the upper limb spasticity assessment are manually performed and, thereby, they could be influenced by the clinician's subjectivity or expertise.

The automation of such traditional methods for spasticity evaluation is an emerging field in neurorehabilitation. Two main classes of systems have been developed for spasticity: (i) passive instruments, which measure the resistance force and/or muscle activity at a given joint which is manually stretched by a clinician, and (ii) active (i.e. robotic) devices which produce a controlled movement of a specific joint at several possible velocities while measuring the resistance force or muscle activity (reviewed in Guo et al., Journal of NeuroEngineering and Rehabilitation (2022) 19:138).

Despite the emergence of these technologies, the penetration of such devices in daily clinical practice is still limited. Devices that can be used for rehabilitation both in a clinical environment and at the patient's home are even more scarce, mainly due to their complex design and lack of portability. In addition, effective upper limb rehabilitation requires specialized physiotherapist guidance, which complicates the rehabilitation process.

Another important consideration is the type of training mode provided by the rehabilitation device. So far there are no prior art rehabilitation devices capable of operating passive, active, and assisted physical therapies for all forms and stages of hand rehabilitation.

Therefore, there remains a need to provide a comprehensive home rehabilitation system providing highly individualized treatment of upper limb function in patients with motor disorders.

## SUMMARY OF THE INVENTION

The invention described herein provides a system for hand rehabilitation exercise, comprising:

a hand exoskeletal device (1) adapted to be worn on the affected hand of a patient, said exoskeletal device (1) comprising a plurality of servomotors (2), each configured to cause the independent motion of a finger of the affected hand;

a glove (13) adapted to be worn on the affected hand of the patient, said glove (13) comprising a plurality of sensors (14, 15, 16, 17, 18, 19) designed to capture the range of motion and/or muscle resistance of one or more fingers of the affected hand, and a microcontroller (20) configured to collect and transmit the raw data from said sensors (14, 15, 16, 17, 18, 19);

a graphical user interface (21) configured to receive the raw data from the microcontroller (20), process said data, and transmit and display the processed data; wherein said graphical user interface (21) further comprises an exercise management environment comprising a plurality of predefined exercise routines, wherein said environment is configured to

provide instructions to the patient for performing one or more of said exercise routines, receive raw data from the microcontroller (20), the data being indicative of the patient's performance of the exercise, and process said data thereby extracting performance metrics; and

an online dashboard (28) adapted to be used by a physician and/or the patient and configured to receive from the graphical user interface (21) the raw data and the processed data and display said data, thereby enabling the patient and/or physician to monitor the performance of the patient.

An advantage of the invention disclosed herein is that it provides a portable system for enhanced treatment and assessment of patients with upper limb spasticity by integrating specialized tests, rehabilitation games and decision support tools, without necessitating a visit to a specialized center or the presence of a physiotherapist. The soft glove with multiple sensors, such as bending and pressure sensors, three-axis gyroscope, accelerometer and magnetometer, allows accurate measurement of fine finger mobility during rehabilitation exercises.

The graphical user interface (GUI) collects streaming data from the sensor glove in real-time, allowing clinicians to monitor patient progress in real-time, for instance via graphs. The GUI also provides the necessary analytics, including scores for each exercise and mean and standard deviation values for each finger. This level of real-time monitoring and analysis is not typically found in traditional rehabilitation equipment, providing a significant advantage to clinicians using the platform. The integration of rehabilitation games into the platform provides a comprehensive way to engage patients in rehabilitation exercises. This gamification approach is not commonly found in traditional rehabilitation equipment, thus providing patients with a motivating rehabilitation experience. The online platform allows clinicians to efficiently store and display processed data, providing a comprehensive overview of patient progress over time. It allows clinicians to easily track patient progress over time and make informed decisions about their rehabilitation.

An additional and related advantage of said system is that it provides new quantitative indicators for the improved assessment and treatment of upper limb spasticity.

Other aspects and benefits of the present invention will become apparent from the detailed description to follow.

BRIEF DESCRIPTION OF THE DRAWINGS

The present invention will now be described with reference to certain embodiments thereof which are illustrated in the accompanying drawings. It should be noted that the accompanying drawings illustrate preferred embodiments of the invention, therefore should not be considered as limiting the scope of the invention.

Fig. 1 is a schematic presentation of an embodiment of the exoskeletal device. (A) is a side view and (B) is a dorsal view.

Fig. 2 depicts a three-dimensional (3D) representation of an embodiment of the exoskeletal device. (A) and (B) shows a three-dimensional representation of an embodiment that does not comprise a hand stabilizing plate. (C) depicts the wrist stabilizing plate and the anchor points of the exoskeletal device according to this embodiment.

Fig. 3 is a schematic presentation of the management of the operation of the exoskeletal device.

Fig. 4 is a presentation of the sensor glove with the integrated sensors and microcontroller.

Fig. 5 is a schematic presentation of different embodiments of the combined exoskeletal device and glove.

Fig. 6 is a schematic presentation of the overall architecture of the system of the present invention.

Fig. 7 depicts the user authentication flow chart when logging in the online dashboard.

Fig. 8 shows examples of the graphic presentations displayed by the GUI. (A) is a flexion graph of the five fingers as presented to the clinician, plotted using the pyqtgraph library. (B) is a projection graph of the pressure on each finger as it is displayed to the clinician using the pyqtgraph library.

Fig. 9 shows the local GUI screen for the Ashworth Scale exercise.

Fig. 10 shows screenshots of (A) the Box & Block and (B) the Sollerman (wallet) rehabilitation games.

DETAILED DESCRIPTION OF THE INVENTION

The present disclosure provides a system for effective rehabilitation of the upper limb that provides highly individualized treatment of upper limb function in patients with motor disorders and can be used both at a physical therapy center and at a patient's home.

The term "exoskeletal device" or "exoskeleton" as used throughout the description and claims refers to a mechanical structure with elements coupled in series, which is adapted to be worn or attached to a user's body and provides structural support and/or rigidity.

The patients for whom this specific aid is intended face a spasticity problem in the upper limb caused by a stroke, multiple sclerosis, brain injuries and various pathologies of the central nervous system such as brain tumors, that result in joint stiffness. As a result of their condition, their fingers are attached to the palmar side of the hand. Thus, the inventors have found that, for the design of an effective home rehabilitation system for the upper limb that would not require the presence of a physiotherapist, the system must possess the functionality of extending the fingers of the patient. At the same time, the system must allow the remote monitoring of the range of motion of the affected limb for evaluating the improvement of the patient. In addition, the system must provide the patient with the impetus to exercise the affected limb on a regular basis.

Thus, the system of the present invention comprises a hand exoskeletal device adapted to be worn on the affected hand of the patient, said exoskeletal device comprising a plurality of servomotors, each configured to cause the independent motion of a finger of the affected hand; a soft glove adapted to be worn on the affected hand of the patient, said glove comprising a plurality of sensors designed to capture the range of motion and/or muscle resistance of the fingers of the affected hand; a user interface that processes the sensor data and further comprises an exercise management environment that includes a plurality of predefined exercise routines; and an online dashboard that enables the patient and/or physician to remotely monitor the results of the exercises performed by the patient.

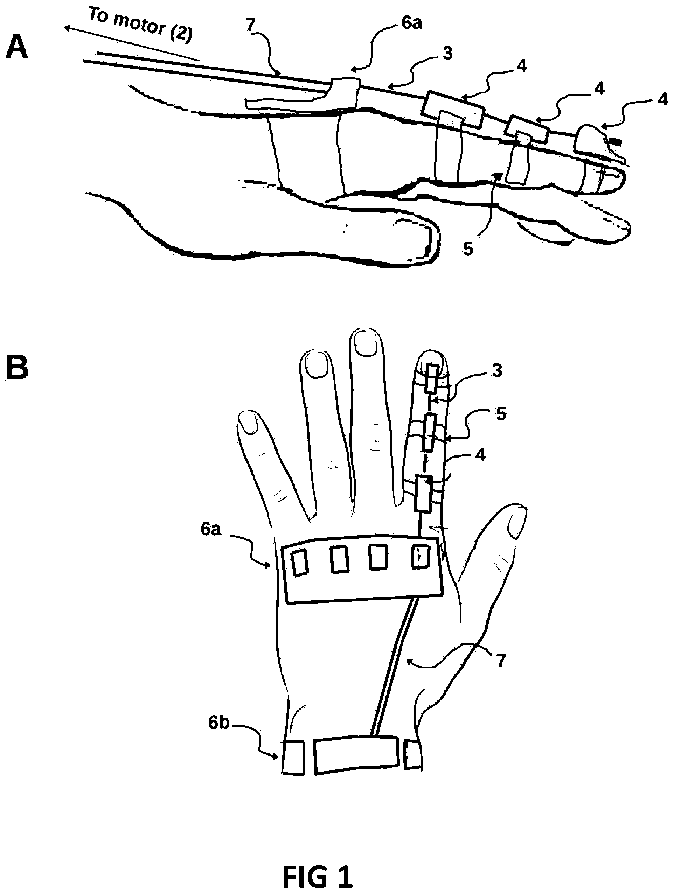

**Exoskeletal device.** Figure 1 presents an embodiment of the exoskeletal device (1) of the present invention. The exoskeletal device (1) is adapted to be worn on the affected hand of the patient. In preferred embodiments, the exoskeletal device (1) is adapted to be mounted directly on the soft glove worn by the patient. Actuation is provided by closed-loop servomotors (2). Exoskeletal hand aids are highly integrated systems, so the choice of actuation (electric motors or pneumatic actuators) is limiting and dictates the choice of energy storage, transmission and drive mechanism. Electric motors (servomotors) are also largely available, reliable and easy to control. Ideally the servomotors used are characterized by high resolution, large rotation angle (about 300 degrees), high speed, and low power consumption, precision in position and speed control, and the ability to operate in torque limit mode and angle limit mode. Preferably, the servomotor (2) is small or miniature-sized. The servomotor (2) ideally has an advanced sensor system and built-in thermostat that allows it to sense the ambient temperature and protect itself from overheating. A suitable servomotor is, as an example, the Dynamixel line of servomotors manufactured by the company Robotis. The exoskeletal device (1) comprises a plurality of servomotors (2). In a preferred embodiment, one servomotor (2) is provided for each of the index finger, middle finger, ring finger and little finger. In this preferred embodiment, the four fingers can be activated independently, therefore, the degree of freedom of the exoskeletal device (1) is four.

The servomotors (2) may be fixedly attached to the outer side of the arm of the patient, preferably on a rigid platform. Less preferred alternatives include placing the servomotors (2) in a carrier bag located at the back of the user, or placing the servomotors (2) on a flat surface near the patient. Servomotors require a method of coupling with the transmission of the hand exoskeleton. In addition, exoskeletal hand aids should be properly aligned with the user's joints. The transmission systems that can be used include cables, rods and gear systems, preferably cables (3). Each servomotor is directly coupled to a reel mechanism comprising a cable (3), such that, when the servomotor (2) moves forward, the reel winds its cable (3) to cause the extension of the finger. When the servomotor (2) moves backward, the reel unwinds its cable (3) to bring the finger back to the initial position, i.e, to bend the finger. The reel mechanism converts the rotary motion into rectilinear such that the cable (3) transfers the kinetic energy of a servomotor (2) to the finger structures thereby causing the independent motion of a finger (or phalanx) of the affected hand. In this embodiment, the servomotors (2) are configured to assist the motion of the finger(s) of the affected hand. The servomotors (2) may additionally be configured to resist the motion of the finger(s) of the affected hand. For instance, the servomotors (2) may apply a tension to the cable (3) such that, when the patient tries to extend a finger he/she needs to overcome the resistance applied by the servomotors (2).

Preferably, the exoskeletal device (1) comprises anchor points (4) adapted to guide the cable (3) along the length of a finger (Figure 1). Preferably, three anchor points (4) are provided per finger, one for each of the distal, middle and proximal phalanges of a finger, as shown in Figure 1. The cross-section of each anchor point (4) is crescent-shaped for automatic alignment with the user's finger. Each anchor point (4) is configured so that a cable (3) can pass through it. The way the anchor points are placed on the finger help distribute the force exerted by the exoskeletal device (1) on the user's hand. Thus, the user is not inconvenienced while using it. Each anchor point (4) is fixedly attached to a finger using a fabric band or other rigid or semi-rigid material, preferably a fabric band (5) that encircles a distal, middle and/or proximal phalanx of a finger. The anchor points (4) are manufactured from any lightweight rigid or semi-rigid material such as plastic or rubber, preferably plastic. Most preferably, the anchor points (4) are manufactured from thermoplastic polyester elastomer such as Z-FLEX, a semi-elastic 3D printing material, resistant to grease and high temperatures. 3D objects printed with this rubber-like material can be squeezed and torn without risk of damage. The anchor points (4) can be attached to the fabric bands (5) by welding or gluing or any other suitable technique, or to the glove (13) by gluing or any other suitable technique. In the embodiment of Figure 1, there are three fabric bands (5) on each of the index finger, middle finger, ring finger and little finger and encircling a distal, middle or proximal phalanx of each finger. Said fabric bands can be adjusted to any finger size by means of an elastic element or a strap such as a Velcro® strap.

In an embodiment, the exoskeletal device (1) comprises one or more stabilizing plates (6a, 6b) that serve the purpose of stabilizing the exoskeletal device on the patient's hand as well as supporting and guiding the cables (3) from the servomotor (2) to the anchor points (4). A stabilizing plate (6a) may be provided on the dorsal side of the wrist of the patient. In the embodiment shown in Figure 1, the stabilizing plate (6a) covers the region of the metacarpal bones. In other embodiments, the stabilizing plate (6a) covers the region of the metacarpal bones and at least partly also the region of the carpal bones. The plate (6a) is attached to a fabric band or other rigid or semi-rigid material, preferably a fabric band (5) that encircles the wrist and can be adapted to fit any wrist size through an elastic element or a strap such as a Velcro® strap. Preferably, the shape of the plate follows the curvature of the patient's wrist. Alternatively or in addition, a stabilizing plate (6b) may be provided on the dorsal side of the hand near the wrist. The plate (6b) is attached to a fabric band or other rigid or semi-rigid material, preferably a fabric band (5) that encircles the hand and can be adapted to fit any hand size through an elastic element or a strap such as a Velcro® strap. The shape of the plate preferably follows the curvature of the patient's hand. The stabilizing plates (6a, 6b) can be manufactured from any lightweight rigid or semi-rigid material such as plastic or rubber, preferably plastic, most preferably thermoplastic polyester elastomer such as Z-FLEX. The plates can be attached to the fabric bands (5) by welding or gluing or any other suitable technique. In addition or alternatively, the plates (6a, 6b) may comprise a pair of openings or a single slot extending generally transversely to the long axis of the plate (6), said openings or slot configured to receive the fabric band (5). For the manufacture of said stabilizing plates (6a, 6b) any suitable technique may be used, preferably 3D printing.

At least part of each cable may be coated by a flexible teflon tube (7) in order to guide the cable (3) from the servomotor (2) to the stabilizing plate(s) (6a, 6b). For instance, a teflon tube may cover the part of the cable (3) extending from the servomotor (2) to the wrist plate (6a).

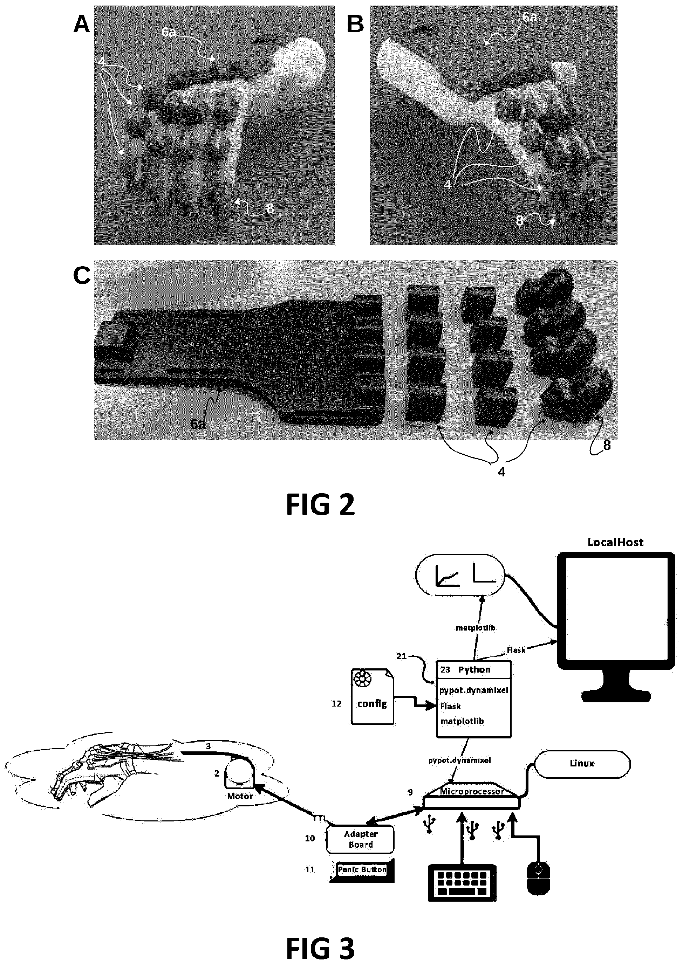

Figure 2 shows a three-dimensional (3D) representation of an embodiment of the exoskeletal device (1). The cables (3) and the fabric bands (5) have been omitted for simplicity. The exoskeletal device (1) of Figure 2 does not comprise a hand stabilizing plate (6b). In this embodiment, the index finger, the middle finger, the ring finger and the little finger, and optionally also the thumb, are provided with a fingertip cap (8) covering the tip of the finger. The fingertip caps (8) are manufactured from any lightweight rigid or semi-rigid material such as plastic or rubber, preferably plastic, most preferably thermoplastic polyester elastomer such as Z-FLEX. Optionally, the fingertip caps (8) are directly attached to the anchor points (4) of the distal phalanx of each finger in order to further stabilize the structure of the exoskeletal device (1). Figure 2C depicts the wrist stabilizing plate (6a) and the anchor points (4) of the exoskeletal device (1) according to this embodiment.

For the construction of the exoskeletal device parts shown in Figure 2, any suitable technique known in the art may be used. Preferably, rapid prototyping technologies such as 3D printing are used for their construction.

To summarize, the design and placement of the anchor points (4) on the hand help distribute the force exerted by the exoskeletal device (1) on the user's hand, thus making the device (1) more comfortable for the user. Another advantage is that the exoskeletal device (1) is portable, and with the power supply and a monitor connection it is possible for the patient to fully operate it. Due to its small size, it can be easily used by any user in any place.

Figure 3 depicts the management of the operation of the exoskeletal device (1). The servomotors (2) are preferably controlled using a microprocessor (single-board computer) (9). The microprocessor (9) is small in size (roughly the size of a regular smartphone) and includes all the necessary components to function, such as a processor, RAM, storage, USB ports and network connections. An adapter board (10) with multiple power ports may additionally be used to connect and power all the motors that actuate the exoskeletal device (1).

All electric functions such as position, velocity, acceleration, force, and other similar attributes, as well as programming functions such as switching operational states, creating and executing motions among other functions can be managed and controlled using a specialized servomotor management software (12) developed by the inventors. The code may be implemented using Python or another suitable programming language. For ease of use and extensibility, a config.json file can be used, which internally declares the information regarding the servomotors (2) used. In this file, the types of motors used as well as the port they are connected to are declared. In addition, for each servomotor (2), its type and ID as well as its baudrate are essential types of information that may be provided in this file. Also, it is possible to initialize the motor's range of motion in degrees, so as to limit its movement to the positions defined. In this way, movements of the fingers that may cause damage are avoided, thus ensuring the safety of the patient. Finally, the number (slots) of the segmented movements of the servomotor (2) up to the final position may also be declared. Setting the value '1' to slots performs a continuous movement to the final position. The use of the config file offers scalability, due to the possibility of declaring different servomotors (2) in terms of their number and model.

*Servomotor rotation angle calculation.* To realize the movement of the fingers of the exoskeletal device (1), the study of their maximum extension angle was required. The reason was to avoid any over-extension of the users' fingers that would lead to injuries. In addition, there was the need for the movement of the fingers to be simultaneous and for this reason the extension speeds of each finger should be adjusted. For the index finger, the angle between the initial (closed) to the final (open) position was measured to be 95.11 degrees. This angle refers to the degree of extension of the index finger and not to the rotation angle of the servomotor (2). So, there should be a way to correlate the rotation angle of the motor (2) with the extension angle of the index finger. For that reason, the equation (I) was used:

$$L = 2 * \pi * r * \left( m/360^o \right) \text{ (I)}$$

where L is the length of the arc of the drum, and m is the servomotor rotation angle. By calculating the rotation angle of the servomotor (2) and knowing its initial position, we can derive the initial and final positions of its movement. The same actions were performed to calculate the rotation angle of the servomotors (2) that drive the rest of the user's fingers. Subsequently, the config.json file is updated with the rotation range of the servomotor (2) that drives each finger. In this way, full extension of the finger is achieved when the servomotor (2) moves to the limits set, correlating the movement of the servomotor (2) with the corresponding driving finger.

*Synchronization of finger movement.* The inventors observed that, when the exoskeletal device (1) moves two fingers (index and middle) at the same speed, the index approached the final position faster than the middle finger. The reason is that the length of the fingers is different, which means each finger has a different arc that it should cover until the final position. Thus, when the user defines a finger extension speed, this speed should concern one finger and then the speed on the other fingers should be proportionally adjusted. To calculate the speed of each finger, the following equation (II) is used:

$$U = \frac{dx}{dt} \text{ (II)}$$

where dx is the length of the arc and dt is the movement time. The speed of each finger should then be correlated with the speed of the index finger using the following equation (III):

$$\frac{U_i}{U_j} = \frac{dx_i}{dx_j} \text{ (III)}$$

where $dx_i$ is the length of the arc of the index finger and $dx_j$ is the length of the arc of the remaining fingers. Based on the results of the above calculations, the simultaneous movement of the fingers from their initial to their final position is achieved.

*Panic button.* Another important consideration for ensuring the safe operation of the exoskeletal device (1) is the provision of a panic button (11) shown in Figure 3, that would enable the patient to interrupt any function of the exoskeletal device (1). One possible solution to this problem would be to provide this capability through software by having a button in the application. However, such a solution would not be reliable because a signal coming from the operating system could interfere with the activation of the panic button. So there should be a solution that cuts off power to all servomotors (2). The solution given to this problem was the addition of a panic button (11) which was connected to the adapter board (10) such that, when activated, immediately stops any operation of the exoskeletal device (1). During the movement of the exoskeletal device (1) the user has the option to select the panic button (11) in order to deactivate the device (1). This was made possible by modifying the way the exoskeletal device (1) is actuated; that is, the exoskeletal device (1) is actuated in short intervals until it reaches the final position, so its operation can be stopped at any time. Thus, a further advantage of the exoskeletal device (1) of the present invention is that it is safe to use.

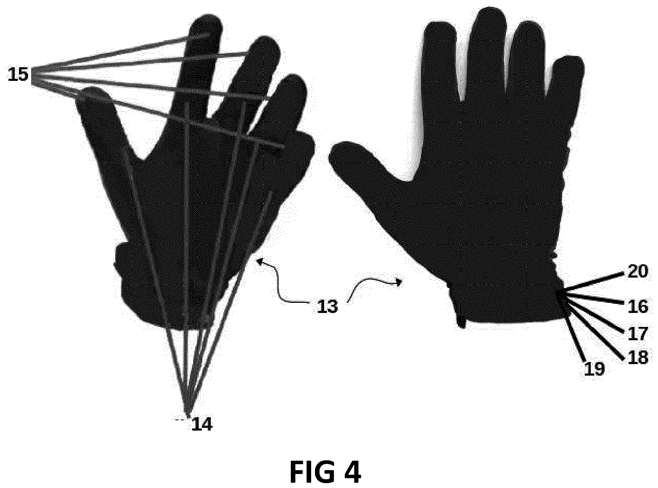

**Sensor glove (13).** Turning to Figure 4, the soft glove (13) integrates a variety of specialized sensors (14, 15, 16, 17, 18, 19) to evaluate the fine motility of the fingers of the affected hand of the patient. The sensors (14, 15, 16, 17, 18, 19) are designed to measure and record various movements and positions of the patient's fingers and wrist during treatment sessions. Thus, the sensors (14, 15, 16, 17, 18, 19) provide accurate and reliable movement or position data that allow the system to monitor and draw useful measurements and conclusions. Said glove (13) may comprise a plurality of bending (flexion) sensors (14), which measure the bending of each finger. Preferably, the glove (13) comprises at least one bending sensor (14) on each finger portion. Alternatively or in addition, the glove (13) may comprise a plurality of pressure sensors (15), which, for instance, measure the pressure exerted on each finger by the physiotherapist during the treatment session. Preferably, the glove (13) comprises at least one pressure sensor (15) located at the tip of each finger portion.

Preferably, the bending sensors (14) of the glove (13) are very sensitive and able to detect finger movements quite satisfactorily. Such sensors allow accurate measurement of finger flexion, which is important for monitoring progress in hand and finger rehabilitation. The glove's pressure sensors (15) are preferably very sensitive and able to detect the amount of force applied to each finger. This information can be used to assess finger strength and identify areas for improvement. The combination of flexion sensors (14) and pressure sensors (15) makes the glove (13) an ideal tool for measuring hand and finger movements.

In a preferred embodiment, said glove (13) also comprises a goniometer (16) that provides the rotation of the wrist (tared quaternion), an accelerometer (17) that provides the linear acceleration of the wrist (linear acceleration), a barometer (18) that provides the altitude at which the wrist is located, and/or a magnetometer (19).

The bending sensors (14) and/or pressure sensors (15) are preferably tethered to a microcontroller (20) integrated in the glove (13). The microcontroller (20) is responsible for the real time wireless or wired transmission of the data collected by the bending (14) and pressure sensors (15). The microcontroller (20) may also be responsible for charging the passive exoskeletal system. Preferably, the microcontroller (20) is placed in the part of the glove (13) that covers the back of the hand. In a preferred embodiment, the goniometer (16), the accelerometer (17), barometer (18) and magnetometer (19) are included in the microcontroller (20).

The glove (13) may additionally comprise one or more Inertial Measurement Unit (IMU) sensors on the wrist and/or fingers. The IMU measures triaxial acceleration and triaxial angular velocity. Each IMU consists of an accelerometer, which can output linear acceleration signals on three axes in space, and a gyroscope, which can output angular velocity signals on three axes in space.

The glove (13) is made, entirely or partially, of flexible material and comprises at least a palmar portion which comes into contact with the palm of the hand, a dorsal portion which comes into contact with the dorsal side of the hand, and in certain embodiment also comprises finger portions for at least an index finger, a middle finger, a ring finger and a little finger, and optionally also for the thumb.

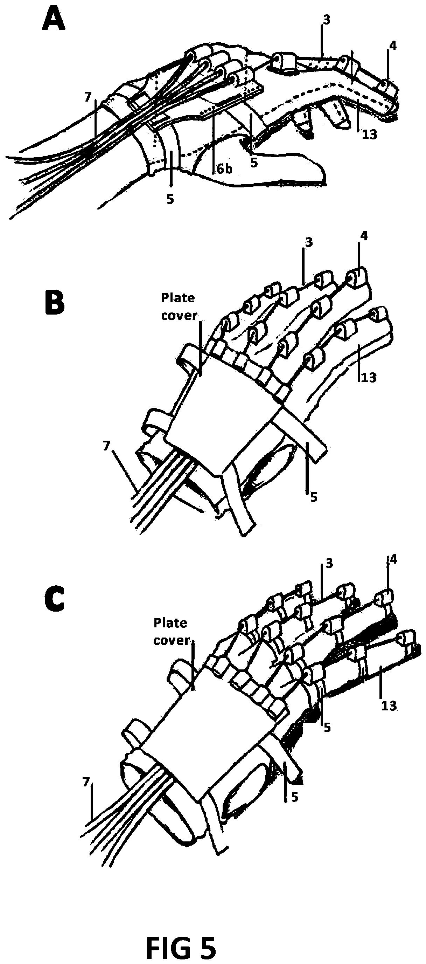

Figure 5 depicts possible embodiments of the combination of the exoskeletal device (1) and the glove (13). In Figure 5A, the glove (13) comprises finger portions for the index finger, the middle finger, the ring finger and the little finger. The anchor points (4) are directly attached to the glove (13) instead of being attached to fabric bands (5). In Figure 5B and 5C, a plate cover is also shown which has been designed to enhance the wearability of the exoskeletal device and to hide the cables leading to the servomotors. The plate cover is preferably made of an elastic fabric or elastic cloth. In Figure 5C, the glove (13) comprises finger portions for the index finger, the middle finger, the ring finger and the little finger. The anchor points (4) are attached to fabric bands (5).

The data collected from the sensors (12, 13, 14, 15, 16, 17) are preferably transmitted wirelessly, however cable connection for wired data transfer is also possible. The wireless transmission preferably uses BLE technology and is capable of transmitting real-time data with a sampling rate of up to 30-35 Hz at full charge. This frequency allows the efficient analysis of the raw data for the extraction of metrics and conclusions.

Overall, the glove (13) is a key component of the system, that provides accurate and reliable data to clinicians and helps improve the clinical status of patients during rehabilitation.

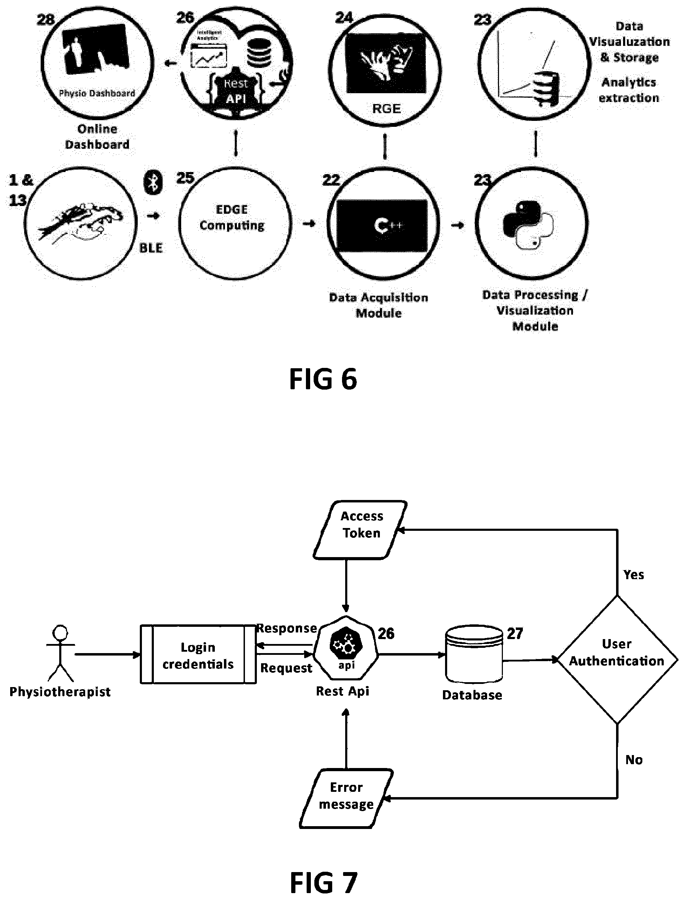

**Graphical User interface (21) developed to support the functionalities of the sensor glove and exoskeletal device.** As schematically shown in Figure 6 depicting the system architecture, an integral part of the rehabilitation system disclosed herein is a local graphical user interface (GUI) (21) for the analysis and presentation of the captured data. This GUI (21) allows for real-time analysis of the data, including the ability to create graphs and charts that help identify trends and patterns. Specifically, using the system described herein, by recording the movements per finger in real time, every movement performed by the user or effected by the exoskeletal device (1) becomes visible, and then the treating clinician can recognize patterns and rate each movement more effectively.

To acquire the sensor data locally in real time, a local server may be used, which receives wirelessly the raw data from the glove (13), wherein the raw data may include pressure, finger bending, hand acceleration, hand motion via the goniometer, to name a few. Using a local server ensures that data can be retrieved and processed immediately, which is crucial for real-time applications. The local server may be developed in C++, the operating system on which this server may be developed is Windows 10 or Linux and a suitable integrated development environment (IDE) is Visual Studio. This allows for a robust and stable platform that can be easily modified and updated as needed. Using an established IDE also ensures that the development process is simplified and the resulting code is of high quality.

The GUI (21) can be divided into data acquisition module (22) and data processing/visualization module (23). For the development of the data processing/visualization module (23) of the GUI (21), the preferred programming language is Python 3.10; preferably, the libraries used to display the data to the user are the PYQT and pyqtgraph libraries. PYQT is a set of Python bindings for the popular application framework Qt, which provides an extensive set of tools for building GUIs. The pyqtgraph library is a fast and efficient data visualization library that provides high-performance graphics and real-time data plotting capabilities. Together, these libraries enable the GUI to display real-time data and provide important tools for data analysis and visualization. By using PyQt to create a GUI, it is possible to display various types of information, including graphs. To achieve this, the technique of widgets and dock areas may be used. Widgets can be used to display various types of information, such as text and buttons. However, in the case of displaying graphs, a widget is preferably created specifically for each graph. This allowed the chart to be displayed in a visually appealing and functional way. The program may also implement dock areas to maintain widgets and graphs. This technique allows the user to reposition or resize them according to their preferences. By using dock areas, the program is able to simultaneously display multiple graphs or other types of information, making it more efficient and user-friendly. Various Python libraries may be used to generate the graphs, such as Matplotlib or Plotly, however, pyqtgraph is the preferred choice for to generating graphs because this library allows for creating many different types of graphs, each with its own unique features and benefits. Overall, the use of the program's widgets and dock areas allows the creation of a flexible and dynamic GUI (21) that can display various types of information, including graphics. By using these tools, the result is a user-friendly GUI (21) program that provides a visually appealing and informative user experience.

The data transfer from one process (C++) to another (Python) in real time may be performed through a multicast forwarding model, such as through the User Datagram Protocol (UDP) which transmits data from one process to another. This method is suitable for real-time applications as it is fast and reliable, ensuring that data can be transmitted quickly and accurately. In addition, the use of multicast forwarding allows efficient distribution of data to multiple recipients, which is useful in applications where data must be shared between multiple users or processes. The process that receives the data through the BLE protocol, has the role of the client in the communication between it and the GUI (21) regarding communication through the UDP protocol for data exchange. To simultaneously achieve these two functions, i.e. acquiring data via BLE protocol and sending them to the GUI (21), multitasking functions can be integrated into the C++ process.

By leveraging the capabilities of the libraries and software that are used to develop the local GUI (21), the resulting software is both powerful and efficient, enabling real-time data acquisition, analysis and visualization. Using these tools also ensures that the software can be easily modified and updated as needed, providing a platform for continuous development and improvement.

In addition to the above capabilities, every user is provided with the capability to control the exoskeletal device (1) through a virtual environment that provides an easy-to-use interface between the back end and front end of the application. A suitable web application framework is Flask, a lightweight framework for developing web applications in the Python language based on the Model-View-Controller (MVC) architecture. It is lightweight and easy to learn, with a simple and flexible structure and a small free code base. This makes it ideal for developing small to medium applications where ease of development and flexibility are important requirements. Flask provides a number of extensions that can be used to add additional functionality to the application, such as authenticating users, connecting to databases, and collecting data from APIs.

Further, it was necessary to create diagrams to display the movements of the fingers, such as graphs, charts and data visualizations in the form of images. Matplotlib is a suitable open source library for the Python programming language which provides a wide range of functions for creating graphs, including line graphs, column graphs, area graphs, scatter graphs, distribution graphs, and more.

An important consideration is that the software that controls the exoskeletal device (1) should be able to start running without user input. For this reason, two script files were implemented, one of which is responsible for starting the software and the second for starting the browser. The first script starts the execution of the implemented python code and at the same time the next script runs in the background to launch the web application. The second script opens the browser and applies its full view. Before starting the second script there is a wait for the first script to have started the program. In the next stage, these two scripts are launched automatically when starting the microprocessor (9), so they were added to the list of programs that are launched when the platform starts. So by connecting the microprocessor (9) to a monitor and power supply, the main page of the implemented application appears in full view.

The integrated architecture for the management of the operation of the exoskeletal device (1) is illustrated in Figure 3 where the parameters of the program and the wiring of the individual devices are shown. In addition, some possible operating scenarios are discussed in detail in the Examples.

As will be more extensively described below, the GUI (21) comprises an exercise management environment integrating a series of exercise routines, designed to train the patient and monitor and extract the relevant metrics. The exercise management environment may further be configured to generate a rehabilitation game environment (24) which uses as input the raw data received from the microcontroller (20).

In addition to the graphical and processing capabilities, the local GUI (21) disclosed herein also acts as an EDGE computer (25), which communicates with the appropriate REST API (26) whose purpose is to efficiently upload data and metrics to an online database (27). This step ensures that the data is easily accessible from anywhere and can be further analyzed.

In summary, the local GUI (21) is a very critical element of the present system, designed to be easy to use for clinicians monitoring the patient's progress. The GUI (21) allows clinicians to see the results of exercises performed by patients and compare them to previous sessions. This allows clinicians to assess patient progress and adjust treatment sessions as needed.

**Integrated exercise routines for upper limb spasticity.** The GUI (21) includes an exercise management environment integrating a series of exercise routines, designed to train the patient and monitor and extract the relevant metrics. The simple interface allows the physician and/or the patient to extract exercise metrics and view key statistics and scores, allowing them to make necessary changes to the exercises. The exercise management environment of the local user interface (21) makes it possible to select the exercises and monitor the results obtained as they are performed by the patient.

In one embodiment, said exercise routines are performed by the patient without requiring the active assistance from the exoskeletal device (1). In this embodiment, the execution of these exercises involves the recording of the data from the sensors (14, 15, 16, 17, 18, 19) and their monitoring on the platform. Preferably, said exercise routine(s) measure one or more of the following parameters among others: a) increase or decrease of muscle tone and b) range of unassisted motion for each finger. The increase or decrease of muscle tone may be measured by assessing muscle resistance to passive stretching, primarily using pressure sensors (15). The range of unassisted motion for each finger may be measured by monitoring finger extension and flexion, finger abduction and adduction, and/or finger opposition, primarily using bending sensors (14).

In an alternative embodiment, the exercise routines integrated in the GUI (21) comprise exercises where at least part of the motion is assisted by the exoskeletal device (1). Said exercises may include the repetitive extension of the fingers up to a maximum desired stretch position. After each extension the patient may return the fingers to their original position or the return of the fingers to the initial position is carried out with the assistance of the exoskeletal device (1). Preferably, said exercise routine(s) measure one or more of the following parameters among others: (a) range of exoskeleton-assisted motion for each finger, b) extend to which the patient managed to return the finger to the original position and c) the return speed of the fingers. The required data such as movement angles of the servomotors, extension and return speed of the fingers may be collected by the servomotor management software (12) (Figure 3).

In a preferred embodiment, the exercise routines integrated in the GUI (21) comprise both exercise routines performed by the patient without requiring the active assistance from the exoskeletal device (1) and exercises where at least part of the motion is assisted by the exoskeletal device (1). In this preferred embodiment, the data captured by the sensors (14, 15, 16, 17, 18, 19) integrated in the glove (13) and/or data originating from the servomotor management software (12) may be used to monitor the progress of the patient.

Alternatively or additionally, the exercise routines integrated in the GUI (21) comprise exercise routines performed by the patient where the exoskeletal device (1) applies resistance to the motion of the finger(s), for at least a part of the motion of said finger(s), in order to provide a more intense exercise and further strengthen the affected hand. Preferably, said exercise routine(s) measure one or more of the following parameters among others: (a) increase or decrease of muscle tone, (b) range of motion for each finger, c) extend to which the patient managed to return the finger to the original position and d) the return speed of the fingers.

The GUI environment can display to the user the score obtained for each exercise using the appropriate options. The user can process and extract the raw data and can also monitor the patient's movements in real time through graphs.

**The rehabilitation game environment (24).** In a preferred embodiment, the GUI (21) also incorporates a rehabilitation game environment (RGE) (24) into its operation, that uses machine vision and augmented reality technologies to receive, interpret and evaluate the spatial information of the user's hand. In this embodiment, the main menu of the GUI is displayed to the user allowing the option to choose between the RGE (24) and the exercise routines established for enhanced patient training. The spatial information regarding the user's hand is provided by the glove (13). Optionally, the spatial information may additionally be derived from a computer camera. The RGE (24) uses the spatial information to generate output signals designed to display appropriate virtual reality images on a display device, such as the display device of a computer or laptop. Preferably, said output signals include a representation of the affected hand and at least one simulated object. Preferably, the representation of the hand is based on the configuration of the glove. The RGE (24) is designed to maximize the performance of the platform even with the use of not specialized and low-cost equipment (computer or laptop) by the end user, physiotherapist and/or patient. The software used to locate and capture the patient's hand may be in C or C++ language.

The rehabilitation games are characterized by a set of input parameters that allow for the customization of each exercise to meet the specific needs of the patient. These input parameters include the selection of the desired hand for conducting the exercise, the adjustment of tolerance values to tailor the application's difficulty, total exercise duration (when applicable), the setting of the size of virtual elements, as well as the desired camera used for the exercise. The extracted performance metrics arising from these rehabilitation game exercises are crucial for evaluation and progress monitoring. The extracted metrics include the identification of the executed exercise, the assignment of a score (relevant for *Sollerman* tests, based on completion and execution quality), the count of blocks moved during the 60-second *Box & Block* Test, total exercise duration, and the hand utilized for the exercise. The rehabilitation games integrated into the system are designed to simulate the five components of full hand functionality, i.e. flexion, extension, adduction, abduction, and circumduction. To the inventors' knowledge, this is not the case for other rehabilitation game solutions existing in the literature. In addition, rehabilitation games engage the patient in a fun and interactive way, while also providing a means of monitoring and evaluating their movements. Games provide an additional level of hands-on training, providing physical therapists with basic functions such as launching them, automatically saving the extracted scores and metrics locally, and uploading the final metrics to the online database (27).

The games display the score in real time to the patient while training but at the same time the extracted metrics are stored locally. The GUI (21) is then responsible for obtaining these scores, for converting them into the appropriate data structure for their effective acceptance by the online dashboard (28), and finally for sending them to the online database (27), using the same access token received during user authentication.

**Online dashboard (28).** The local GUI (21) can efficiently transmit both processed and raw data to the online database (27) for display on the online dashboard (28). After each exercise or rehabilitation game, the local tool processes the data and sends it to the online database (27) using POST requests through the REST API (26). This ensures that both the raw data and the extracted metrics are converted into appropriate data structures that the REST API (26) accepts. To avoid data loss, the local platform also stores the raw data locally in a hierarchical structure in CSV files for each exercise performed. This allows the data to be stored locally for later analysis and ensures that no data is lost during transmission to the online database (27). In addition, local storage of the raw data allows for future analysis of the data and the ability to develop new metrics based on the data collected.

The present invention also provides a method for performing self-rehabilitation of upper limb spasticity using the system disclosed herein. The data transfer process commences when the glove (13) is activated and begins transferring sensor data to a local EDGE computer (25). The computer runs the software responsible for collecting data from the glove (13), preferably using Bluetooth Low Energy technology. The data acquisition module (22), such as a C++ module, imports the data and then sends it to the data processing/visualization module of the GUI (21) shown in Figure 6, which is a Python module, for further processing. This software is also responsible for displaying the data in real-time graphs and storing them locally.

After processing and storing the data locally, the last step is to send it to the cloud. Data is sent to a REST API (26) whose purpose is to store the data in an online database (27). This step ensures that the data is easily accessible from anywhere and can be further analyzed.

In addition to data management, the local user interface on the EDGE computer (25) has a critical function in exercise control. The physical therapist can choose which exercise to perform and start or stop virtual reality games through the user interface. The interface is simple to use and provides a wide range of options to control exercises.

Apart from the ability to exercise control, the user interface also allows the user to extract exercise metrics and view key statistics and results. This feature allows the user to track progress and make necessary changes to exercise routines. The interface is designed to be user-friendly and easy to navigate, making it accessible to a wide range of users.

In conclusion, data management and exercise control procedures are critical elements of the system. The EDGE computer (25) runs software that collects and processes data from the glove (13) and sends it to the cloud. The user interface provides an easy-to-use platform for exercising control and monitoring progress. The simple interface allows the user to extract exercise metrics and view key statistics and scores, allowing them to make necessary changes to their exercise routines.

EXAMPLES

Hereinafter, the present invention is described in more detail with reference to examples and comparative examples. It will be apparent to one of ordinary skill in the art that these examples are for illustrative purposes only and should not be construed as limiting or altering the scope of the present invention.

Example 1 - Communication with the online dashboard (28)

When launching the GUI (21), clinicians are asked to enter their personal credentials to enter the online dashboard (28) (Figure 7). The backend of the platform automatically checks if the correct credentials have been entered. This takes place through a request made using the REST API (26), which in turn communicates with the online database (27) and checks if the current user is registered. If so, the local platform allows access to the user and at the same time receives a token, with which the local tool is able to transfer the appropriate data (raw and processed) to the online database (27). The user is then able to select the patient code corresponding to the current patient to be trained with the system.

After logging in, the patient is able to send some data to the online database (27) using the local GUI (21). When the patient completes an exercise routine or rehabilitation game, the program sends a request to the API with the data and metrics payload as well as the access token received during authentication. The API then validates the access token to ensure it matches the patient's stored token and grants access to the patient's account. If the access token is valid, the API accepts the data payload and adds it to the patient's database record. This process ensures that only authorized users can submit data to the online database (27), and the API validates the access token to ensure that the data comes from a legitimate source.

After the end of each selected exercise routine or rehabilitation game, the extracted data is sent to the online dashboard (28) where the physical therapist can connect to monitor the progress of the connected patients through the monitoring of data. When the physician logs in, a list of registered patients is displayed. This list is displayed in the form of codes which correspond to the patients that the doctor has created on the online dashboard (28) so that the extracted data can be sent to the corresponding patient. This is how the general monitoring of the patient's progress is achieved through the online dashboard (28).

The matching of local patients with the online dashboard (28) is done using the REST API (26) and the access token acquired during authentication. Finally, these codes are used for the local storage of the data in the appropriate patient codes with a hierarchical structure. Then by selecting exercises the physician can see the exercises that were successfully performed for that particular patient.

Example 2 - Using the GUI (21) to display the captured data.

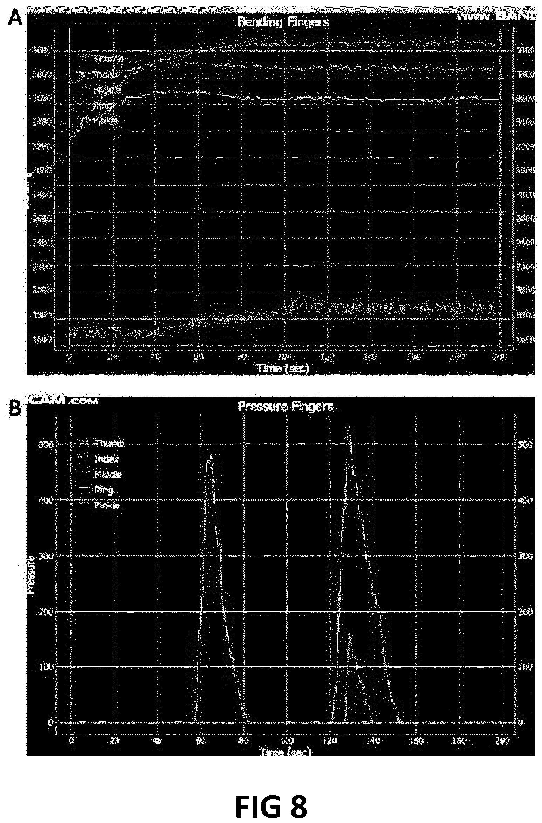

Figure 8A is a flexion graph of the five fingers as presented to the clinician, plotted using the pyqtgraph library. In the graph above the fingers are in the initial equilibrium position which in the case of patients with fine mobility problems is the full flexion of the fingers. As the fingers are stretched by performing the prescribed clinical exercises, the graphs shift accordingly in real time. The vertical axis expresses the flexion in units specific to the exoskeletal device (1) while the horizontal axis expresses the exercise execution time. The sampling frequency is approximately 30 Hz and is sufficient for the clinician to draw appropriate conclusions and rate the patient's effort accordingly.

A projection graph of the pressure on each finger as it is displayed to the clinician using the pyqtgraph library is presented in Figure 8B. In this graph, the difference in the shape of the waveforms to those in Figure 8A is immediately noticeable. Specifically, in this graph it becomes apparent that pressure is applied to the index finger, then there is a pause, and then pressure is applied to two fingers, the index finger and the thumb. No waveform is detected and therefore no pressure is exerted on the remaining fingers. The vertical axis expresses pressure in sensor-specific units of the passive exoskeleton, while the horizontal axis expresses time. The frequency is again 30 Hz and is sufficient for the clinician to draw useful conclusions.

Example 3 - Using the integrated exercise management environment.

When launching the GUI (21), the patient may select one of the offered exercises. Some of the exercises that may be integrated into the online platform include: (i) the Ashworth Scale (AS), which is the most universally accepted clinical tool used to measure the increase of muscle tone, (ii) the Passive Range of Motion (PROM), (iii) the Active Range of Motion (AROM), and (iv) Free Session [Meseguer-Henarejos AB et al. Eur J Phys Rehabil Med. 54(4):576-590, 2018; Hosseini ZS et al. J Caring Sci. 8(1):39-44, 2019]. The physical therapist in charge can select the appropriate exercise to train the patient with and monitor and extract the relevant metrics. The exercises are then presented as they are viewed in the GUI (21) and the physiotherapist can choose to initiate the exercises of interest for each session.

When an exercise is selected, the GUI (21) displays the main objective of the exercise in the form of information text and illustrates the graphs of the data of interest for each finger in real time. The user can also obtain the appropriate score for each exercise using the appropriate options. Thus, the user can start displaying and recording the data to monitor the patient's movements in real time through graphs from sensors specialized for the assessment of upper limb spasticity. The user may also process and extract the raw data in order to...

*A. Ashworth Scale (AS).* The Ashworth Scale is a tool used by clinicians to assess muscle spasticity in people with hand injuries undergoing rehabilitation. To measure the AS, pressure sensors (15) are used to record the level of resistance exerted by the patient in units specific to the sensor. A large score corresponds to difficulty in finger extension which translates into increased pressure on the fingers.

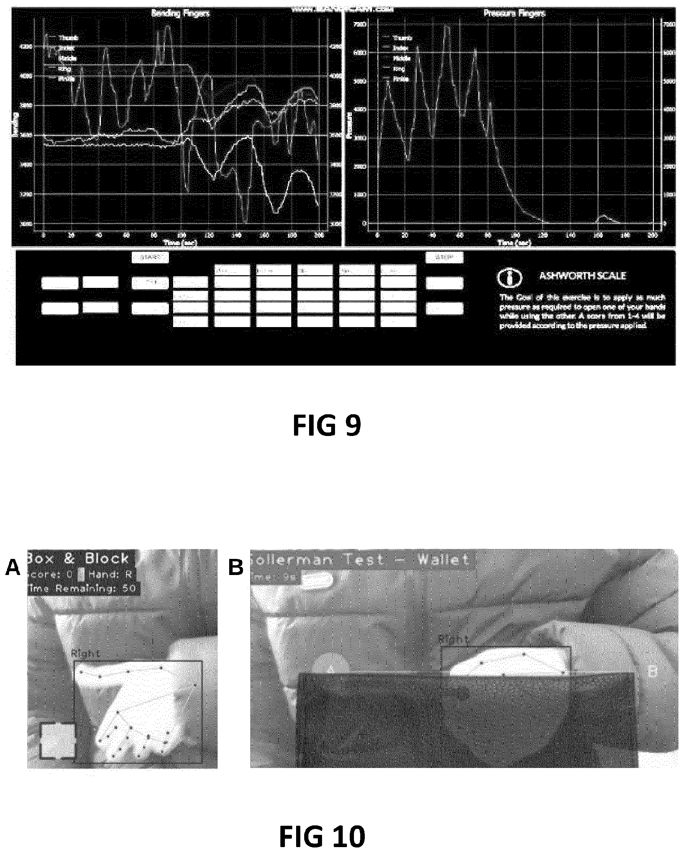

Figure 9 presents the GUI screen for the AS exercise. It is shown that the data of the bending (left graph) (14) and pressure (right graph) (15) sensors can be derived in real time from the five fingers of the upper limb. A different color is used to illustrate the waveform of each finger to facilitate clinicians in the assessment of the relevant data. When the button "STOP" is selected by the user, the categorization of the patient according to the AS is depicted at the lower level of the window, as well as the mean and standard deviation values for each finger separately.

*B. Passive ROM (PROM).* PROM exercises are commonly used when patients are unable to perform active movements due to pain, weakness, or neurological deficits. By passively moving the fingers, the therapist can help maintain joint mobility, prevent muscle spasms, and promote healing of soft tissue structures. The therapist will carefully monitor the patient's response to the exercise and adjust the intensity and duration of the movements as needed to avoid causing further damage or discomfort.

To measure the passive range of motion as it results from the exercise of external forces (e.g. physical therapist), finger bending sensors (14) are used which are capable of recording a time series of data expressing the extension and flexion of each finger during duration of the exercise. Based again on the theory of this specific exercise, it becomes clear that by calculating the range of motion of each finger, a good picture can be presented of the course of the patient's rehabilitation and of his improvement/deterioration. Specifically, large ranges of motion indicate improvement in spasticity of motion while smaller ranges of motion indicate difficulty in finger extension/flexion.

*C. Active ROM (AROM).* In the Active range of motion (ROM) exercises, the patient actively moves his/her fingers through their full range of motion without assistance. To derive the necessary score but also to monitor patient progress in relation to the active range of motion, the bending sensors (14) of the glove (13) are again used. When the patient himself tries to extend his/her fingers, the measurement of the bending sensors (14) is activated and it becomes clear that his/her attempt is successful if he/she achieves large ranges of motion overall as an average for all fingers, while smaller ranges of motion, as shown in the GUI (21), correspond to difficulty in extending or flexing the fingers. At the same time, the flexion graphs that are updated in real time show any changes in the range of motion for each finger achieved by the patient and give the physiotherapist in charge a complete picture of the execution of each exercise.

When the PROM or AROM exercises are chosen by the user, both the data of the bending (14) and the pressure (15) sensors are available in the GUI screen in real time. The data that can be extracted for PROM and AROM are the degrees of motion, while the mean and standard deviation values are also depicted using a graphical representation.

*4. Free Session.* In addition to the above predefined clinical exercise routines, the graphical user interface allows for a free session, through which both the physical therapist in charge and the patient can familiarize themselves with the operation of the exoskeletal device (1). In particular, the physical therapist will be able to monitor the response of the graphs in real time to understand the way in which the data transmission is carried out and to better understand the analysis and quality offered in terms of the raw data. At the same time, the clinical physiotherapist has the possibility to extract some basic statistical metrics such as average values and typical deviations concerning the bending (14) and pressure (15) sensors per finger, and thus become more familiar with the measurements recorded by the GUI (21).

It should be mentioned that the signal values provided by the bending (14) and pressure (15) sensors are not based on any known system. To that end, many experiments were carried out by the inventors in collaboration with clinical physical therapists for the effective mapping of these values with the extracted scores of the exercises.

Example 4 - Rehabilitation games

The most common physical therapy/medical protocol for assessing the patient's condition, known as the Box and Block test, has been implemented and integrated into the virtual environment of the present invention. Also, the corresponding protocol of Sollerman's therapeutic exercises (Sollerman Hand Function Test) has been considered, which aims to enhance the patient's daily life skills (e.g. opening-closing a wallet zipper, unlocking a door, opening a jar, lifting plate from the table, etc.) through the proper training. More specifically, the virtual games for opening-closing a wallet zipper and unlocking a door have also been integrated into the platform: in *Box & Block,* the patient tries to move the bricks from one end of the screen to the other; in *Sollerman (wallet),* a wallet appears on the screen and the patient tries to move the zipper from point A to point B. Figure 10 presents screenshots from the *Box and Block* test (Figure 10A) and *Sollerman (wallet)* game (Figure 10B). Other Sollerman games include *Key* (which simulates unlocking a door), *Jar* (simulates unscrewing a jar lid), *Screwdriver* (simulates the handling of a screwdriver) and *Draw* (simulates writing with pen on paper).

Example 5 - Managing the assisted finger movement web application framework

Using the web application framework, the patient has the option to choose among four available modes of movement effected by the exoskeletal device (1). The first three modes concern the repetitive extension of the fingers up to the maximum stretch position and the fourth gives the option of repeating the movement up to a point chosen by the user. The point should be within the limits of the possible finger extension, otherwise the upper extension point is set to the one specified by the config.json file.

The developed application fully manages assisted finger movement through different features available. Mode 1 is the extension of each finger to the highest point and then the patient is able to return the fingers to their original position. As mentioned above due to the nature of the disease, the return of the fingers takes place spontaneously without their control. In Mode 1, an interaction environment has been created for the input of parameters by the user to start its operation. The speed of finger extension to the upper limit should be stated as well as the maximum number of repetitions of the movement. Start is then selected and the servomotors start moving. Once a finger extension is performed, the patient is responsible for returning the fingers to their original position. Then selecting the Next Round button moves to the next iteration of the move. This gives the patient the necessary time to restore the fingers. Upon completion of all iterations, the movement angles of each servomotor in degrees are displayed on the user interface.

An additional feature provided by the application is the display of the servomotor motion diagram. When the user selects the Finish button, the application proceeds to its display. In the diagram displayed, the x axis refers to time and the y-axis refers to the position of the servomotor (in degrees). The range of the values in the y axis depends on the maximum rotation angle of the specific servomotor. The upper peaks of the graph show the position of the index finger at maximum extension and the lower peaks the position the index finger reached when the patient returned moved the finger back towards the original position.

Two more interaction interfaces (Modes 2 and 3) with similar functionality can be implemented. The difference with Mode 1 is that the return of the fingers to the initial position is carried out with the help of the servomotor. The user inputs the extension speed of the fingers together with the speed of their return to the initial position. The two modes differ in that, in Mode 2 the user is responsible for starting each repetition of the move via a Next Round button which appears on the interface, while in Mode 3 the user sets the waiting time between successive repetitions.

Mode 4 allows the user to set as a parameter the maximum angle of extension of the desired finger. In the event that the extension angle does not exceed the maximum extension point of the finger, the movement is repeated. Otherwise, the movement is reaches the maximum point of extension that was previously calculated and stored in the config.json file. In Mode 4 as in Mode 2, the user is responsible for starting each round by selecting Next Round. The difference in Mode 4 is that the servomotor moves iteratively until the finger approaches the position set by the user.

Example 6 - Performance Measurements

The accuracy of the system disclosed herein was assessed by comparing the analytics and scores produced by the system to those resulting from manual assessment by clinical physiotherapists. Preliminary results from a clinical study have been derived from seven stroke patients evaluated, first from a clinician and then with the use of the system disclosed herein, using the AS score. The mean age was 67.2 years (SD = 10.62), and the mean time since the stroke was 4.85 months (SD = 1.64). The mean AS score for the clinicians' measurements was 1.85 (SD = 0.83), whereas the mean measurement score for the system of the present invention was 1.71 (SD = 0.45). AS score data proceeded for statistical analysis using an independent t-test, one-tailed distribution, and two-sample unequal variance. There were no statistically significant differences between clinician and system measurements (p-value=0.36).

The results showed that the system disclosed herein is quite accurate, with a low rate of false positives and false negatives. Ashworth scale scores produced by the system were consistent with the scores derived from the manual assessment, with a high level of agreement between the two methods. This suggests that said system will be effective in recording the level of spasticity in patients with neurological conditions, which is a critical factor in determining the success of their rehabilitation.

Furthermore, the system also demonstrated a high level of consistency in the analytics and scores produced across multiple sessions performed by physiotherapists to familiarize them with the system, indicating its reliability and making it suitable for monitoring patients' progress with their course of time. This was particularly evident in the passive and active ROM exercises, where the platform consistently produced accurate and precise scores within the target range of 0-90 degrees for as many times as the physical therapists tested.

**Claims**

1. System for hand rehabilitation exercise, comprising:

a hand exoskeletal device (1) adapted to be worn on the affected hand of a patient, said exoskeletal device (1) comprising a plurality of servomotors (2), each configured to cause the independent motion of a finger of the affected hand;
a glove (13) adapted to be worn on the affected hand of the patient, said glove (13) comprising

a plurality of sensors (14, 15, 16, 17, 18, 19) designed to capture the range of motion and/or muscle resistance of one or more fingers of the affected hand, and
a microcontroller (20) configured to collect and transmit the raw data from said sensors (14, 15, 16, 17, 18, 19);

a graphical user interface (21) configured to receive the raw data from the microcontroller (20),
process said data, and transmit and display the processed data; wherein said graphical user interface (21) further comprises an exercise management environment comprising a plurality of predefined exercise routines, wherein said environment is configured to

provide instructions to the patient for performing one or more of said exercise routines, receive raw data from the microcontroller (20), the data being indicative of the patient's performance of the exercise, and process said data thereby extracting performance metrics; and

an online dashboard (28) adapted to be used by a physician and/or the patient and configured to receive from the graphical user interface (21) the raw data and the processed data, and to display said data, thereby enabling the patient and/or physician to monitor the performance of the patient.

2. The system according to claim 1 or 2, wherein said exercise management environment is further configured to generate a rehabilitation game environment (24) for interacting with the patient and for providing visual feedback, wherein said rehabilitation game environment (24) uses as input the raw data received from the microcontroller (20) to extract performance metrics.

3. The system according to claim 2, wherein said system further comprises a camera, arranged such that, the image data captured by the camera are also used as input by the rehabilitation game environment (24).

4. The system according to anyone of claims 1 to 3, wherein the exercise management environment is adapted so that the physician can select or modify the exercise routine(s) and/or rehabilitation game(s) to be performed by the patient, based on the extracted performance metrics.

5. The system according to anyone of the preceding claims, wherein said graphical user interface (21) is further configured to receive from the servomotors raw data that include the movement angles of the servomotors and/or extension and return speed of the fingers; process said data; and transmit and display the processed data.

6. The system according to anyone of the preceding claims, wherein said plurality of sensors comprises bending sensors (14) and/or pressure sensors (15).

7. The system according to claim 6, wherein the microcontroller (20) comprises a goniometer (16) that provides the rotation of the wrist, an accelerometer (17) that provides the linear acceleration of the wrist, a barometer (18) which provides the altitude at which the wrist is located, and/or a magnetometer (19).

8. The system according to anyone of the preceding claims, wherein each servomotor (2) is connected to one or more cables (3) arranged to transfer the kinetic energy of the servomotor (2) to a finger, thereby causing the independent motion of the finger of the affected hand.

9. The system according to claim 8, wherein said exoskeletal device (1) comprises a plurality of anchor points (4) fixedly attached to a finger and adapted to guide the cable (3) along the length of the finger.

10. The system according to claim 8 or 9, wherein said the exoskeletal device (1) comprises one or more stabilizing plates (6a, 6b) adapted to stabilize the exoskeletal device (1) on the patient's hand and to support and guide the cables (3) from the servomotors (2) to the fingers of the affected hand.

11. Method for evaluation and rehabilitation exercising of the hand, comprising the following steps:

   providing a patient with a glove (13) to be worn on the affected hand of said patient, the glove (13) comprising a plurality of sensors (14, 15, 16, 17, 18, 19) designed to capture the range of motion and/or muscle resistance of one or more fingers of the affected hand;
   providing the patient with a hand exoskeletal device (1) to be mounted directly on the glove (13), said device (1) comprising a plurality of servomotors (2) each configured to cause the independent motion of a finger of the affected hand;
activating said glove (13) and exoskeletal device (1) thereby enabling the patient to perform one or more exoskeletal device (1)-assisted or -unassisted predefined exercise routines;
collecting sensor data from said glove in real time;
using a graphical user interface (21) to process said data in order to extract performance metrics; thereby enabling the patient and/or a physician to monitor the performance of the patient.

12. The method according to claim 11, further comprising the step of using the graphical user interface (21) to generate a rehabilitation game environment (24) for interacting with the patient and for providing visual feedback, wherein said rehabilitation game environment (24) uses as input the sensor data collected to extract performance metrics.

13. The method according to claim 11 or 12, further comprising the step of selecting or modifying the exercise routine(s) and/or rehabilitation game(s) to be performed by the patient based on the extracted performance metrics.

**FIG 1**

**FIG 2**

**FIG 3**

**FIG 4**

**FIG 5**

**FIG 6**

**FIG 7**

FIG 8

**FIG 9**

**FIG 10**

Europäisches
Patentamt

European
Patent Office

Office européen
des brevets

## EUROPEAN SEARCH REPORT

**Application Number**

EP 23 20 8205

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | POTSIKA VASSILIKI ET AL: "An integrated rehabilitation system for the upper limb spasticity assessment and treatment: the Rehabotics passive exoskeletal system", 2023 45TH ANNUAL INTERNATIONAL CONFERENCE OF THE IEEE ENGINEERING IN MEDICINE & BIOLOGY SOCIETY (EMBC), IEEE, 24 July 2023 (2023-07-24), pages 1-4, XP034488846, DOI: 10.1109/EMBC40787.2023.10340161 [retrieved on 2023-12-11] * the whole document * | 1-11 | INV. A61H1/02 G06F3/01 B25J13/02 |
| Y | DRAGUSANU MIHAI ET AL: "Design, Development, and Control of a Hand/Wrist Exoskeleton for Rehabilitation and Training", IEEE TRANSACTIONS ON ROBOTICS, IEEE SERVICE CENTER, PISCATAWAY, NJ, US, vol. 38, no. 3, 1 June 2022 (2022-06-01), pages 1472-1488, XP011910546, ISSN: 1552-3098, DOI: 10.1109/TRO.2022.3172510 [retrieved on 2022-05-20] * the whole document * | 1-11 | |
| Y | FR 3 062 058 A1 (BOUTELDJA MALIK [FR]) 27 July 2018 (2018-07-27) * the whole document * | 1,2,4,6, 8-11 | TECHNICAL FIELDS SEARCHED (IPC) G06F A61H A61B A63B B25J |
| A | WO 2016/174083 A1 (BIOSERVO TECH AB [SE]) 3 November 2016 (2016-11-03) * paragraph [0018] - paragraph [0019] * * paragraph [0021] * * paragraph [0028] * * paragraph [0032] * | 1,6,8-11 | |

The present search report has been drawn up for all claims

| Place of search | Date of completion of the search | Examiner |
|---|---|---|
| Munich | 19 April 2024 | Tejada Biarge, Diego |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 8205

This annex lists the patent family members relating to the patent documents cited in the above-mentioned European search report.
The members are as contained in the European Patent Office EDP file on
The European Patent Office is in no way liable for these particulars which are merely given for the purpose of information.

19-04-2024

| Patent document cited in search report | Publication date | Patent family member(s) | Publication date |
|---|---|---|---|
| FR 3062058 A1 | 27-07-2018 | NONE | |
| WO 2016174083 A1 | 03-11-2016 | EP 3288721 A1 | 07-03-2018 |
| | | ES 2748975 T3 | 18-03-2020 |
| | | JP 6755885 B2 | 16-09-2020 |
| | | JP 2018517575 A | 05-07-2018 |
| | | KR 20180019512 A | 26-02-2018 |
| | | US 2018116310 A1 | 03-05-2018 |
| | | WO 2016174083 A1 | 03-11-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **GUO et al.** *Journal of NeuroEngineering and Rehabilitation*, 2022, vol. 19, 138
- **MESEGUER-HENAREJOS AB et al.** *Eur J Phys Rehabil Med.*, 2018, vol. 54 (4), 576-590
- **HOSSEINI ZS et al.** *J Caring Sci.*, 2019, vol. 8 (1), 39-44